# EUROPEAN PATENT SPECIFICATION

(11) **EP 2 304 051 B1**
(45) Date of publication and mention of the grant of the patent: **11.02.2015**
(21) Application number: 08789467.1
(22) Date of filing: 28.07.2008
(51) Int. Cl.: C12Q 1/68

(54) **HUMAN MENA ISOFORM SERVE AS MARKER FOR SENSITIVITY TO EGFR INHIBITION IN HUMAN CANCER CELLS**
MENSCHLICHE MENA-ISOFORM ALS MARKER FÜR DIE EMPFINDLICHKEIT GEGENÜBER EGFR-HEMMUNG BEI MENSCHLICHEN KREBSZELLEN
ISOFORMES DU MENA HUMAIN SERVANT DE MARQUEURS DE LA SENSIBILITÉ À L'INHIBITION DE L'EGFR DANS DES CELLULES HUMAINES CANCÉREUSES

(30) Priority: 12.06.2008 IT RM20080310
(43) Date of publication of application: 06.04.2011
(73) Proprietor: Istituti Fisioterapici Ospitalieri (Ifo)- Istituto Regina Elena Per Lo Studio e La Cura Dei Tumori, 00144 Roma (IT)
(72) Inventor: NISTICO', Paola, I-00144 Roma (IT); DI MODUGNO, Francesca, I-00144 Roma (IT); PINO, Maria Simona, I-00144 Roma (IT)
(74) Representative: Predazzi, Valentina
(86) International application number: PCT/IB2008/053023
(87) International publication number: WO 2009/150494

(56) References cited:
- DI MODUGNO FRANCESCA ET AL: "Molecular cloning of hMena (ENAH) and its splice variant hMena(+11a): Epidermal growth factor increases their expression and stimulates hMena(+11a) phosphorylation in breast cancer cell lines" CANCER RESEARCH, vol. 67, no. 6, March 2007 (2007-03), pages 2657-2665, XP002510455 ISSN: 0008-5472
- PINO MARIA S ET AL: "Epithelial to mesenchymal transition (EMT) and hMena splice variants expression are determinant of EGFR tyrosine kinase inhibitors (TKI) sensitivity in pancreatic adenocarcinoma (PDAC) cell lines." PROCEEDINGS OF THE AMERICAN ASSOCIATION FOR CANCER RESEARCH ANNUAL MEETING, vol. 48, April 2007 (2007-04), page 713, XP001537056 & 98TH ANNUAL MEETING OF THE AMERICAN-ASSOCIATION-FOR-CANCER-RESEARCH; LOS ANGELES, CA, USA; APRIL 14 -18, 2007 ISSN: 0197-016X
- DI MODUGNO FRANCESCA ET AL: "hMena+11a is an epithelial-restricted hMena isoform which is phosphorylated along the pathway of ErbB tyrosine kinase receptors." PROCEEDINGS OF THE AMERICAN ASSOCIATION FOR CANCER RESEARCH ANNUAL MEETING, vol. 48, April 2007 (2007-04), page 905, XP002510457 & 98TH ANNUAL MEETING OF THE AMERICAN-ASSOCIATION-FOR-CANCER-RESEARCH; LOS ANGELES, CA, USA; APRIL 14 -18, 2007 ISSN: 0197-016X

## Description

### BACKGROUND OF THE INVENTION

The epidermal growth factor receptor (EGFR) plays a central role in cell life by controlling processes such as growth or proliferation. This receptor is commonly over expressed in a number of epithelial malignancies and its up regulation is often associated with an aggressive phenotype of the tumour.

Amplification and/or over expression of the epidermal growth factor receptor (EGFR) and its ligands have frequently been observed in a variety of human malignancies including pancreatic cancer in which the receptor is over expressed in about 30-60% of the cases being associated with disease progression and resistance to conventional therapy and adverse prognosis.

Thus, targeting of EGFR has represented a very promising challenge in oncology, and drugs raised against this receptor have been investigated as potential anti tumour agents some of them, such as tyrosine kinase inhibitors and antibodies, are already in use and result in a synergistic anti tumour effect with chemotherapeutic agents or with radiotherapy. Therefore, several ongoing studies include EGFR-directed therapy either alone or in combination with chemoradiotherapy for cancer disease.

By way of example, cetuximab and panitumumab are monoclonal anti-bodies targeting EGFR. In some clinical trials, these drugs bring a survival benefit with acceptable toxicity. In Western, bevacizumab, cetuximab and panitumumab have been already approved for patients with unresectable colorectal cancer, and used in clinical practice. In the last few years EGFR has shown to be a good target for therapy of several tumours including pancreatic carcinoma and some inhibitors of the receptor's activity are now available for therapy such as erlotinib (Tarceva™; Roche, Basel, Switzerland and OSI, Melville, NY). Erlotinib is an orally bio available small molecule inhibitor of the EGFR tyrosine kinase domain that recently became the first targeted therapy clinically approved medicament, in combination with gemcitabine, for the treatment of locally advanced or metastatic pancreatic cancer (Moore MJ, Goldstein D, Hamm J et al. Erlotinib Plus Gemcitabine Compared With Gemcitabine Alone in Patients With Advanced Pancreatic Cancer: A Phase III Trial of the National Cancer Institute of Canada Clinical Trials Group. J Clin Oncol 2007;25:1960-6.). However, although statistically significant, the improvement in survival was small. In fact, a different level of sensitivity to EGFR-inhibition therapy was reported in experimental models, calling for better prospective selection of those patients who might benefit of the above therapy. Said selection can be achieved by identifying reliable predictive marker (s) that identify EGFR-dependent tumors (Moasser MM, Basso A, Averbuch SD, Rosen N. The Tyrosine Kinase Inhibitor ZD1839 ("Iressa") Inhibits HER2-driven Signalling and Suppresses the Growth of HER2-overexpressing Tumor Cells. Cancer Res 2001;61:7184-8; Engelman JA, Janne PA, Mermel C et al. ErbB-3 mediates phosphoinositide 3-kinase activity in gefitinib-sensitive non-small cell lung cancer cell lines. Proceedings of the National Academy of Sciences 2005;102:3788-93; Thomson S, Buck E, Petti F et al. Epithelial to Mesenchymal Transition Is a Determinant of Sensitivity of Non-Small-Cell Lung Carcinoma Cell Lines and Xenografts to Epidermal Growth Factor Receptor Inhibition. Cancer Res 2005;65:9455-62). The role of EGFR mutation as a principal mechanism in conferring sensitivity to EGFR inhibitors has become controversial and moreover, mutations predictive of outcome have not been found in pancreatic cancer (Lynch TJ, Bell DW, Sordella R et al. Activating Mutations in the Epidermal Growth Factor Receptor Underlying Responsiveness of Non-Small-Cell Lung Cancer to Gefitinib. N Engl J Med 2004;350:2129-39; Shepherd FA, Rodrigues Pereira J, Ciuleanu T et al. Erlotinib in Previously Treated Non-Small-Cell Lung Cancer. N Engl J Med 2005;353:123-32; Tsao M-S, Sakurada A, Cutz J-C et al. Erlotinib in Lung Cancer: Molecular and Clinical Predictors of Outcome. N Engl J Med 2005;353:133-44).
Mechanisms aside from mutation of the EGFR tyrosine kinase domain must therefore dictate drug sensitivity.

Increasing evidence suggests that during tumour progression, malignant cells exploit critical developmental and tissue remodelling programs, often promoting a plastic phenotype referred to as an epithelial to mesenchymal transition (EMT). This process is characterized by a disassembly of cell adherens junctions and the acquisition of a more motile and invasive phenotype through a significant reorganization of the actin cytoskeleton. Of interest, recent reports have shown that in NSCLC (Non-small Cell Lung Cancer) an EMT-like transition is predictive of erlotinib resistance *in vitro* and *in vivo.*

Enabled/vasodilator-stimulated phosphoprotein (Ena/VASP) are key regulatory molecules controlling cell shape, movement and actin organization at cadherin adhesion contacts, which are frequently affected following malignant transformation. We have shown that human Mena (hMena), a member of the Ena/VASP family, is over expressed in human breast tumors, and a splice variant termed hMena^{+11a} was recently isolated from a breast cancer cell line with an epithelial phenotype. Of interest, experimental data suggest that hMena couples tyrosine kinase signalling to the actin cytoskeleton (Di Modugno F, DeMonte L, Balsamo M et al. Molecular cloning of hMena (ENAH) and its splice variant hMena+11a: epidermal growth factor increases their expression and stimulates hMena+11a phosphorylation in breast cancer cell lines. Cancer Res 2007;67:2657-2665).
Di Modugno et al. "Molecular Cloning of hMena (ENAH) and Its Splice Variant hMena+11a: Epidermal Growth Factor Increases Their Expression and Stimulates hMena+11a Phosphorylation in Breast Cancer Cell Lines" Cancer Research 67, 2657-2665, March 15, 2007" discloses the molecular cloning of the splice variant hMena + 11a and a pan hMena antibody capable of distinguishing all forms of hMena but not the specific hMena +11a.
Pino Maria et al. "Epithelial to mesenchymal transitition (EMT) and hMena splice variants expression are determinant of EGFR tyrosine kinase inhibitors (TKI) sensitivity in panchreatic adenocarcinoma (PDAC) cell lines". Proceeding of the American association for cancer research annual meeting vol. 48, April 2007 (2007-04), page 713 discloses that some unspecified hMena splice variants expression are determinant for assessing the EGFR tyrosine kinase inhibitors (TKI) sensitivity in panchreatic adenocarcinoma.
Di Modugno et al. "hMena+11 is an epithelial-restricted hMena isoform which is phosphorylated along the pathway of ErbB tyrosine kinase receptor" Proceeding of the American association for cancer research annual meeting vol. 48, April 2007 (2007-04), page 905 discloses hMena +11a as a promising biomarker for the identification of different breast cancer.

Hence, although new therapeutics have been identified for the treatment of pancreatic carcinoma and other tumours, said new medicaments that act by inhibiting EGFR activity are not effective on all pancreatic carcinomas and on all kind of tumours. It is very important to know the efficacy and safety of these drugs for the clinical practice. It is hence evident that it would be useful to identify molecular markers that could indicate whether a certain therapy will be effective or not, thus avoiding to submit patients to ineffective treatments and, at the same time, pointing out those patients for which treatment with EGFR inhibitors could be of use.

Scope of the invention is to identify predictive markers that could help clinicians to prospectively select cancer patients who are eligible to positively respond to the EGFR inhibitors/antagonists therapy.

### SUMMARY OF THE INVENTION

The expression of hMena and hMena^{+11a} was first characterized in a panel of human pancreatic cancer cell lines showing heterogeneity in responsiveness to the TKI inhibitor erlotinib. Whereas in other normal tissues hMena expression has been reported at low or no detectable levels, hMena was detected in all the pancreatic tumour cell lines tested, in a human pancreatic ductal epithelial cell line (HPDE) as well as in pancreatic tissue, primary and metastatic tumours.

Surprisingly, it has been found out by the present inventors that the expression of hMena^{+11a}, an isoform specific to cell lines that display an epithelial phenotype, was mainly restricted to cancer cell lines that were E-cadherin positive and negative for expression of vimentin and N-cadherin. Notably, these cell lines also displayed constitutive activation of the EGFR pathway and significant sensitivity to erlotinib. Collectively these data lead us to find that hMena^{+11a} is not only a marker of an epithelial phenotype, but its expression is also able to identify cancer cells that are utilizing the EGFR to drive proliferation, rendering them sensitive to EGFR-specific TKIs. On the contrary, the hMena^{+11a} isoform was found to be not expressed in mesenchymal, erlotinib-resistant cancer cell lines.

Therefore, the inventors have shown that hMena acts as a mediator of the EGFR signalling pathway and significantly modulates the growth of pancreatic cancer cell lines dependent on EGFR signalling. These cell lines, which display an epithelial phenotype are erlotinib-sensitive and are selectively characterized by the presence of hMena^{+11a} isoform. As a whole, the results of the inventor's study highlight the expression of hMena/hMena^{+11a} as a predictive marker of *in vitro* or *in vivo* response to anti-cancer treatment with EGFR inhibitor drugs.

Accordingly, a first object of the present invention is a method for discriminating between sensitive and resistant tumours to a treatment with EGFR inhibitor drugs comprising: *in vitro* testing whether tumour material expresses the hMena^{+11a} splicing variant of hMena, the tumour positive to said testing being sensitive to the treatment. The tumour is an epithelial tumour selected frompancreatic, breast, colorectal, ovarian, lung cancer, and the tumour material being selected from a specimen of tumour tissue, tumour cells or in blood circulating tumour cells.

According to an embodiment of the invention the method the testing of the hMena^{+11a} expression is made by detecting the hMena^{+11a} isoform protein using one or more labelled antibody or labelled fragment thereof specific for said hMena^{+11a} isoform. In an alternative embodiment of the invention the testing of the hMena^{+11a} expression is made by detecting the hMena^{+11a} mature transcription products by hybridisation with a labelled probe specific for hMena^{+11a}.

In a still alternative embodiment of the invention the testing of the hMena^{+11a} expression is made by detecting the hMena^{+11a} mature transcription products by PCR amplification of the hMena^{+11a} variant.

A second object of the invention is the use of: a) at least an antibody or fragment thereof specific for hMena^{+11a} splicing isoform, said antibody or fragments capable of being detected; b) one or more labelled probes specific for hMena^{+11a} mature transcription products; c) two or more primers amplifying the hMena^{+11a} variant for identifying tumours and/or blood circulating tumour cells sensitive to a treatment with EGFR inhibitor drugs, wherein the rumour an epithelial tumour i.e. breast, colorectal, ovarian, pancreatic, lung cancer.

Additional object of the invention is a method for detecting the presence of in blood circulating tumour cells (CTC) of tumours sensitive to treatment with EGFR inhibitors/antagonists in a tumour-affected patient, comprising assessing whether the hMena^{+11a} splicing variant of hMena is expressed in the blood sample.

The present invention allows to determine whether an epithelial tumour, i.e. breast, colorectal, ovarian, pancreatic, lung cancer, is resistant or sensitive to treatment with EGFR inhibitors/antagonists hence offering the advantage of predicting whether a patient affected by said tumour will be eligible to benefit from a treatment with EGFR inhibitors/antagonists, so avoiding the risk of subjecting not responsive debilitate patients to useless treatment.

### BRIEF DESCRIPTION OF THE FIGURES

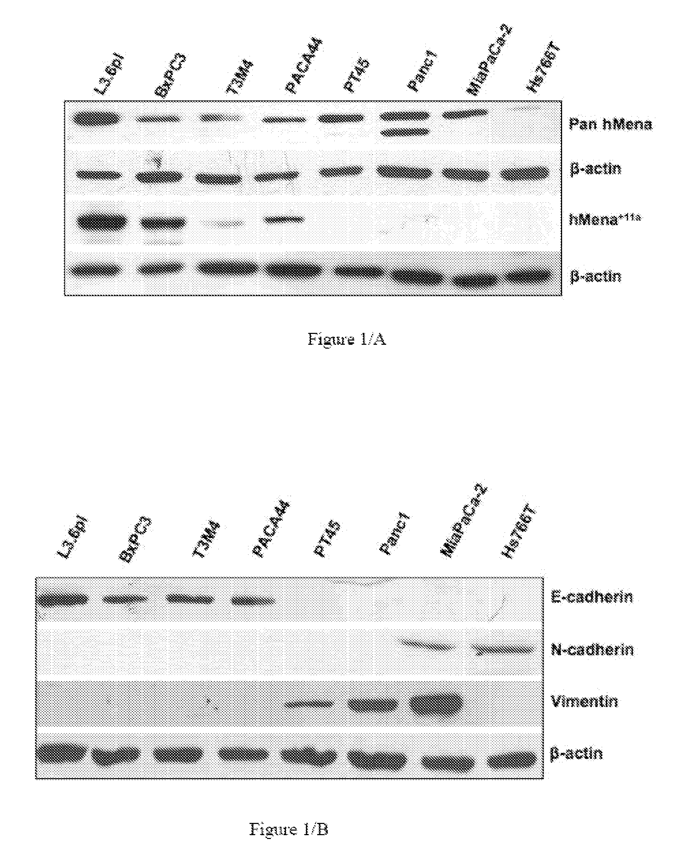

Figure **1****.** hMena and **hMena**^{+11**a**} isoform expression in pancreatic **cancer-**derived cell **lines.** Panel A: protein extracts (25 µg) from pancreatic tumour cell lines were immunoblotted with an anti-hMena CKLK1 antibody (Di Modugno F, Bronzi G, Scanlan MJ, Del Bello D, Cascioli S, Venturo I, Botti C, Nicotra MR, Mottolese M, Natali PG, Santoni A, Jager E, Nisticò P. Human Mena protein, a serex-defined antigen overexpressed in breast cancer eliciting both humoral and CD8+ T-cell immune response. Int J Cancer. 2004 May 10;109(6):909-18) and an anti-hMena^{+11a} specific antibody. As loading control the same blots were probed with an anti-actin monoclonal antibody.
   Panel B, the expression of epithelial (E-cadherin) and mesenchymal (N-cadherin and vimentin) proteins in the panel of pancreatic tumour cell lines was evaluated by Western blot analysis. Actin was used as a protein loading control.

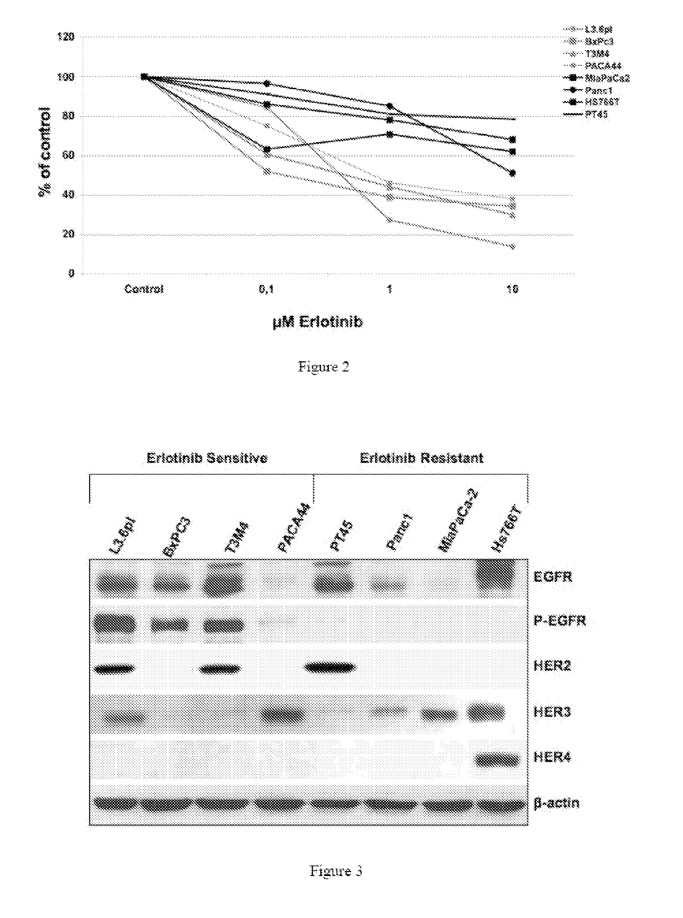

**Figure 2****. Concentration-dependent effects of erlotinib on cell proliferation.** The effects of increasing concentrations of erlotinib (0.1 to 10 µmol/L) on cell proliferation were assessed by measuring the intracellular ATP content using the Vialight assay. Results are expressed as percentage inhibition of ATP incorporation relative to untreated cells. Erlotinib-sensitive cells (gray) and -resistant cells (black). Representative results of at least three separate experiments are shown. In the raw data, the standard error (SE) did not exceed 10%.
**Figure 3****. Sensitivity to erlotinib does not correlate with HER family members expression in pancreatic cancer cell lines.** Protein extracts (100 µg) from erlotinib sensitive pancreatic cancer (L3.6pl, BxPC3, T3M4 and PACA44) and resistant (PT45, Panc1, MiaPaCa-2 and Hs766T) cell lines, were analyzed by Western blot analysis using an anti EGFR and phospho EGFR, HER2, HER3 and HER4 antibodies. As loading control the same blots were probed with an anti-actin monoclonal antibody.

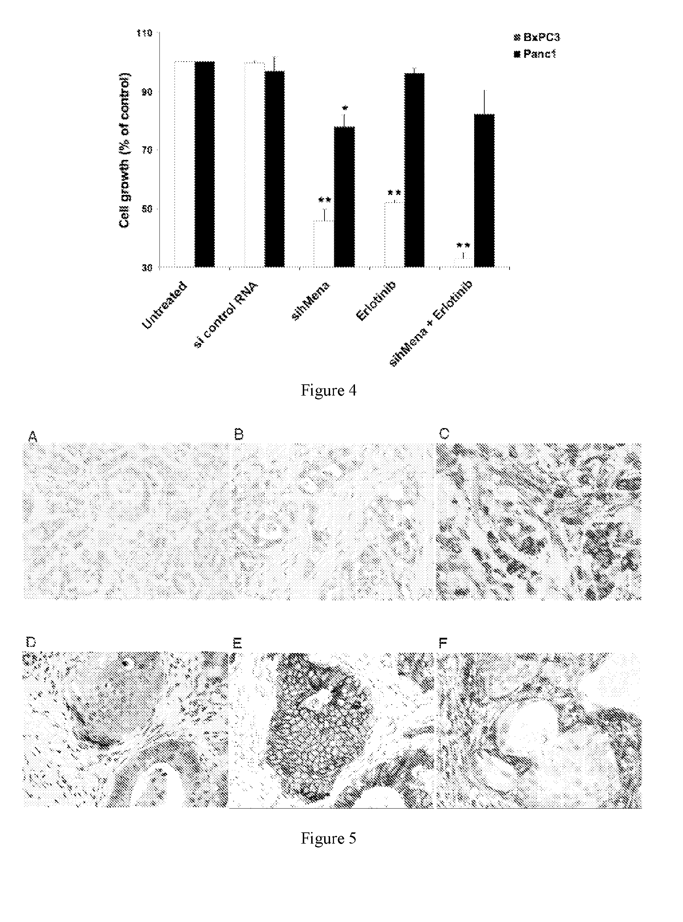

**Figure 4****. hMena knock-down reduces proliferation and phosphorylation of AKT and MAPK in the hMena^{+11a} positive, erlotinib-sensitive pancreatic cancer cell lines.** Silencing of hMena reduces the baseline growth rate in BxPC3 compared with the growth rate of untransfected cells and cells transfected with non specific siRNA (45% vs 100%, *P*<0.01). Not that in the Panc1 cell line hMena knock-down was significantly less efficient in reducing proliferation (78% vs 100%, P<0.05). Furthermore, a dramatic decrease in cell proliferation was observed in the hMena-silenced BxPC3 cells treated with erlotinib at a concentration of 0.1 µM/L for 24 h. Proliferation assays were conducted 72 h after the siRNA transfection.
**Figure 5****. Evaluation of hMena +11A expression in pancreatic carcinomas by immunohistochemistry.** The absence of immunoreactivity towards hMena+11a (A) is associated with (B) the loss of E-cadherin expression and positive results for (C) vimentin whether the expression of hMena+11a (D) (score 3+) is associated with (E) homogeneous expression of E-cadherin and (F) absence of vimentin expression (magnification 40X).

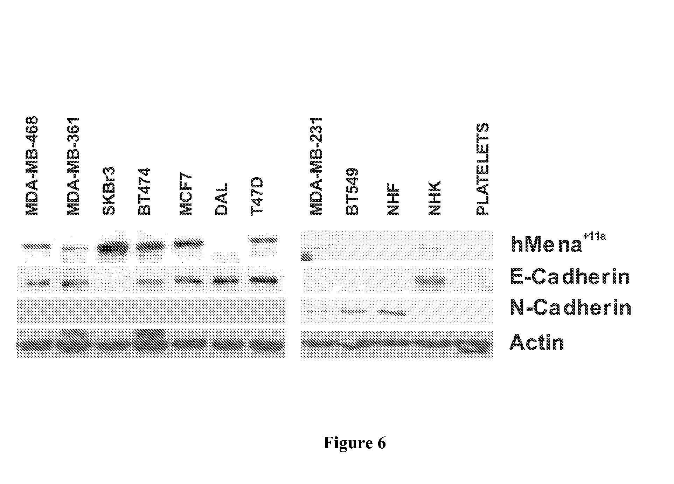

**Figure 6****.** Western Blot analysis of epithelial breast cancer cell lines (left panel), invasive breast cancer cell lines, normal human fibroblast (NHF), normal human keratinocytes (NHK), and human platelets (right panel) with the indicated antibodies.

### DETAILED DESCRIPTION OF THE INVENTION

**Human Mena^{+11a}** (F. Di Modugno et al. Cancer Res. 67:(6), March 15, 2007) is a splicing variant of hMena encoding the corresponding hMena^{+11a} protein isoform. This splicing variant contains the additional *exon 11a* of 63 nucleotides, corresponding to an additional peptide of 21 amino acids characterizing the isoform. The hMena^{+11a}mRNA has sequence SEQ ID NO:1, the exon 11a mRNA has sequence SEQ ID NO:2 and the encoded peptide has sequences SEQ ID NO:3.

Amongst the **tumour types** that are at present treated with drugs having epithelial growth factor receptors (EGFR) inhibiting or antagonizing activity there are breast,colorectal, ovarian, pancreatic, lung cancer.

By EGFR inhibitor or antagonist it is herein intended any substance that acts by inhibiting, antagonising or blocking EGF receptors such as specific immunoligands, antibodies, kinase inhibitors and others. Typically, known **EGFR inhibiting drugs** are commercial products such as Tarceva or Erlotinib or other medicaments having the same effect.

The discriminating method according to the present invention can be carried out either *in vivo* or *in vitro.* However, for the purpose of the present invention, the method is carried out *in vitro,* by testing whether tumour material expresses the hMena^{+11a} splicing variant, the tumour positive to said testing being sensitive to the treatment.

**Tumour materials** within the meaning of the invention are specimens of tumour, isolated tumour cells or in blood-free circulating tumour cells collected from samples of body liquids such as blood, serum, lymphatic liquid. Tumour specimen are obtained from the patients by standard techniques such as biopsy or aspiration.

The **testing** of tumour material for hMena^{+11a} expression can be performed either by detecting the expressed isoform protein or by detecting the mature transcription products, namely the spliced transcript mRNA of hMena^{+11a} or alternatively the cDNA obtained by reverse transcription of the mRNA.

Such detection can be performed with techniques for detecting either nucleotide material or peptide material well known in the art. Method suitable for the detection are either qualitative or quantitative methods. However, since the very presence of hMena^{+11a} expression already represents the positive answer indicating sensitivity and responsiveness to the anti-cancer treatment, it will not be necessary to limit the detection procedure to techniques for quantification of the level of expression.

For *in vitro* procedures the expression of the hMena^{+11a} splicing variant can be detected by methods known to the skilled person including immunologic techniques such as western blot, immunhistochemical techniques, hybridisation techniques, immunofluorescence techniques, and other known techniques capable of detecting a specific protein or a portion thereof or a specific RNA or cDNA or portions thereof, including, in this case, at least part of the 11a exon i.e. Northern Blot, amplification by PCR or RT-PCR.

The detection on the sample under investigation can be carried out by using a reagent selectively binding to the hMena^{+11a} splicing isoform (coded by the nucleic acid of SEQ ID No 1). Said reagent can be a specific antibody or a fragment thereof, wherein said antibody or fragment thereof specifically forms complexes with the hMena^{+11a} splicing variant isoform. The fragment according to the present description can be, without being limited to it, a F(ab')2 fragment, a Fab' fragment or single chained antibodies.

The antibodies according to the description include monoclonal and/or polyclonal antibodies showing a specific binding to the hMena^{+11a} splicing variant isoform.

The polyclonal antibodies can be obtained by the skilled person following standard techniques, by way of example, by immunising rabbits, or mice, or other suitable animals, with the peptide encoded by exon 11a or an antigenic portion thereof, said peptide being coded by SEQ ID No 3, while said antigenic portion being a fragment, such as, by way of example, the peptide of SEQ ID No 4. An effective polyclonal antibody for carrying out the methods herein described can be obtained by immunising rabbits with the peptide having Seq Id No 4, conjugated with KLH.

The immune sera thus obtained can be purified by affinity chromatography for example on Sepharose resin CnBr conjugated with the immunogenic peptide. The Anti- hMena^{+11a} thus obtained will specifically recognise the hMena^{+11a} isoform without cross reacting with the other hMena isoforms in western blot and immunohistochemical experiments (see figure 5). Western blot analysis and immunohistochemical analysis can be carried out following standard protocols known to the skilled person and described in several laboratory manuals.

Suitable monoclonal antibodies can be manufactured by ablating the spleen of animals immunised with the above described immunogens and by fusing the spleen cells with mieloma cells in order to form an hybridome that, once cultured, will produce the monoclonal antibody. The antibody can be an IgA, IgD, IgE, IgG or IgM. The IgA antibody can be an IgA1 or IgA2, the IgG can be an IgG1, IgG2, IgG2a, IgG2b, IgG3 or IgG4. Also a combination of antibodies subtypes can be used. The antibody can be a human, mouse, goat or rabbit antibody. The antibodies can be humanised by using standard techniques of recombinant DNA known to the skilled person.

The reagent binding in a specific way hMena^{+11a} can be complexed with a detectable marker. The labelling can be carried out through many know techniques including but not limited to, peroxydase, chemoluminescence, radioactive markers. The marker can be a non radioactive or a fluorescent marker such as biotin, fluorescein (FITC), acridine, carboxy-X-rhodamine and others known to the skilled person, that can be detected by means if fluorescence techniques or other image analysis techniques.

Alternatively, the marker can be a radioisotope emitting radiations such as, by way of example ³⁵S, ³²P, o ³H. The emitted radioactivity can be detected by means of know standard techniques, such as, by way of example, scintigraphy.

The expression of the hMena^{+11a} splicing variant can be detected also by nucleic acids hybridisation analysis using one or more probes hybridising in a specific manner with the mRNA sequence coding for hMena^{+11a}(Seq Id No 1). In order to avoid cross hybridisation with other hMena isoforms the probe suitable for the present description will have to include in its sequence the nucleic acids coding for exon 11a (indicated in Seq Id No 2) or a portion thereof. The length of the probe can be of a length suitable for Hybridisation, known to the skilled person, suitable length can be between, by way of example, 500 and 10 nucleotides, such as 400, 300, 200, 150, 100, 63, 50, 30, 20, 10 nucleotides and could be, by way of example, of SEQ ID N2 or a fragment thereof or a fragment of SEQ ID No 1 comprising at least a part of SEQ ID No 2.

The probe can be prepared by means of various different techniques known to the skilled person, such as automatic synthesis of suitable oligonucleotides or digestion by restriction enzymes of the nucleotide sequence of hMena^{+11a}, or by cloning the cDNA of hMena^{+11a} or a portion thereof comprising at least a part of Seq Id No 2 in suitable vectors (i.e. plasmids). The sequence of interest can be, e.g. cloned by amplifying hMena^{+11a} from mRNA with the primers having Seq Id No 10 and Seq Id No 11 and subsequently by sub cloning the amplified sequence in suitable vectors known by the skilled person. Cloning techniques are considered today as common knowledge for the geneticist, said techniques being available in detail on any laboratory manual for molecular genetics. A combination of more than one probe can be used for the detection of hMena^{+11a} expression. The detection of expression of the molecule of interest can be carried out by Northern Blot analysis, or by Real-time-PCR or by PCR on cDNA.

Suitable primers for RT-PCR are primers selected downhill or uphill the sequence coding for exon 11a or including a portion of said exon depicted in Seq Id No 2. Suitable primers for RT-PCR according to the description are the primers having Seq id No 5 and Seq Id No 6.

The probes can be labelled with detectable markers such as radioisotopes such ³⁵S, ³²P, o ³H or with biotin, fluorescein (FITC), acridine, carboxy-X-rhodamine or Cy class fluorophores (e.g. Cy2, Cy3, Cy5, Cy7) and others known to the skilled person.

hMena^{+11a} expression can also be detected by means of PCR on cDNA obtained by reverse transcription of the mRNA extracted from the samples tested using primers that allow the amplification of a region of hMena^{+11a} cDNA comprising exon 11a thus allowing a specific amplification of the cDNA of interest. Suitable primers are, by way of example, primers of Seq ID No 7, Seq Id No 8 and Seq Id No 9. It is clear that the skilled person, knowing the sequence of the hMena^{+11a} cDNA and of exon 11a (respectively, Seq Id No 1 and Seq Id No 2) will be capable, without use of inventive skill, to design further primers or probes suitable for carrying out the method of the description.

hMena^{+11a} expression can be concomitant to the expression of other known markers of epithelial phenotype such as E-cadherin and to the absence of expression of markers of mesenchymal phenotype such as N-Cadherin and vimentin (cfr. Fig. 5). The detection of said proteins is known in the art.

hMena^{+11a} expression can be concomitant with the constitutive expression of EGFR in active form, presenting auto-phosphorylation sites in phosphporylated form.

In particular, for the purpose of discriminating between responsive and resistant tumours to EGFR inhibitor drugs, the expression can be normalized by comparison to proteins the expression of which is uniform in sensitive and resistant tumours, such as the isoform of hMena (88kDa) or the VASP protein.

The method of the invention can also be carried out on blood samples of patients affected by EGFR inhibitors/antagonists sensitive tumours, in order to detect the presence of circulating tumour cells (CTC). Said cells, in fact, can be detected in the peripheral blood of patients with a variety of solid cancers. Because of their very low frequency, these tumour cells are not easily detected using conventional cytology methods. In the past decade, numerous groups have attempted to detect CTC of solid malignancies using the highly sensitive reverse transcriptase polymerase chain reaction, which has been shown to be superior to conventional techniques. However, the biological significance of CTC and the therapeutic relevance of their detection are still debated. The method herein described, allows identifying CTC of tumours that are sensitive to EGFR inhibitor/antagonist treatment thus enabling also researchers to further investigate the potential of identification and molecular characterization of the subset of CTC responsible for metastasis development. The prognostic value of CTC would provide clinicians with a unique tool for better stratification of patients' risk and provide basic researchers with a new target for the development of novel therapeutic approaches. The method of the invention advantageously enables the detection of a large group of CTC of different tumours. When aiming to the detection of CTC, the method of the invention will be carried out on the particulte portion of blood sampled deriving from patients affected by a tumour sensitive to EGFR inhibitor drug treatment, in order to avoid, when using antibodies for the detection, to detect free circulating antigens.

Advantageously, the embodiments of the method based on the detection of nucleic acids can be carried out on full blood sample tests.

The present description also discloses Kits for carrying out the methods of the invention.

The kits for carrying out the methods herein described can comprise one or more anti-hMena+11a antibodies, monoclonal or polyclonal, the antibodies being labelled as described above. In one embodiment the antibodies can be antibodies raised for the epitope depicted in SEQ ID No 3 or 4.

The antibody or antibodies of the kit can be in one or more vial, in master solutions or ready-to use solutions, they can be labelled in any of the above described manners. Depending on the labelling selected, the kit of the invention can further comprise the reagents suitable for the detection of the antibody. All the labelling options cited are well-known in the art and the detection protocols and reagents are also well known to the skilled person.

Alternatively, the kit can comprise one or more specific probe to be hybridised either to mRNA or to cDNA and specifically identifying the hMena+11a splicing variant. When the probe will have to hybridise to mRNA, it is clear that the probe will comprise at least a part of the antisense region of the mRNA coding for hMena+11a, said region comprising at least a part of exon 11a. The probes can be labelled as indicated above and probes for use on RNA can be particularly useful for Northern Blot analysis.

When due to hybridise on cDNA the probes can be designed in order to hybridise to the sense or to the antisense strand, and will comprise a region of at least a part of the sequence coding for hMena+11a cDNA said region comprising at least a part of Seq Id No. 2.

As before, the labelling can be any suitable labelling indicated above. The kit may hence further comprise reagents suitable for the detection of the labelled probe. The following examples are intended to merely exemplify certain modes to carry out the invention without limiting the same to them.

The advantages involved in the present invention are evident. The invention enables the skilled person to predict whether or not a cancer therapy including treatments anti-EGFR is possibly helpful for a tumour-affected patient. It is well known that most anti-cancer treatments are highly aggressive to the organism and debilitate the patient. It is hence very useful and advantageous to provide a method and means to predict the effectiveness of an anti-tumoral therapy as it enables the medical team to predict whether a patient would be eligible to have advantages from a certain treatment (in the present case an anti-EGFR treatment as described above). On reverse, if there is no potential benefit, as the tumour examined proves not responsive, a useless and debilitating treatment on the patient can be avoided. On the other hand, the detection of the sensitivity to EGFR inhibitors/antagonists, will allow a targeted and effective treatment, putting also the patient in the positive position of knowing that the tumour treated is sensitive to the drugs used.

It is also clear that a further advantage of the description lies in the fact that certain expensive therapies according to the description, can be avoided when the tumour to be treated can be identified as resistant to the said therapies.

### EXPERIMENTAL SECTION

**Reagents.** Erlotinib was kindly provided by Roche. A stock solution was prepared in DMSO and stored at -20°C. Recombinant human EGF was purchased from Promega (Madison, WI). The antibodies used for Western blot analyses were from the following sources: rabbit anti-total AKT, rabbit anti-pAKT (Ser⁴⁷³), rabbit anti-total mitogen-activated protein kinase (MAPK, p42/44), and mouse anti-pMAPK (Thr²⁰²-Tyr²⁰⁴) were from Cell Signaling Technology (Beverly, MA); rabbit anti-pEGFR (Tyr¹⁰⁶⁸) was from Biosource (Camarillo, CA); rabbit anti-total EGFR, rabbit anti- HER2, mouse anti-HER3 and rabbit anti-HER4 were from Santa Cruz; mouse anti-E-Cadherin from BD Biosciences; mouse anti-N-Cadherin and mouse anti-Vimentin from DakoCytomation (Glostrup, Denmark), and mouse anti-βactin was from Sigma-Aldrich (Poole, United Kingdom). We previously developed the anti-Mena rabbit polyclonal antibody (CKLK1) against the 20 amino acid C-ter peptide of hMena.

**Cell lines and culture conditions.** The following cell lines were purchased from the American Type Culture Collection (Rockville, MD): BxPC3, Panc1, MiaPaCa-2, Hs766T. The L3.6pl human pancreatic cancer cell line was kindly provided by Dr I. J. Fidler (The University of Texas M.D. Anderson Cancer Center, Houston, TX). The T3M4, PACA44, and PT45 cell lines were kindly provided by Dr. F. Velotti (Tuscia University, Viterbo, Italy). All cell lines were maintained in RPMI 1640 (Life Technologies, Inc., Gaithersburg, MD). The medium was supplemented with 10% fetal bovine serum (FBS; Life Technologies), L-glutamine (Bio Whittaker, Rockland, ME), and antibiotics (penicillin/streptomycin; Bio Whittaker). Adherent monolayer cultures were incubated at 37°C in a mixture of 5% CO₂ and 95% air.

**Cell treatments.** Cells were grown in six-well plates to confluence in RPMI supplemented with 10% fetal bovine serum. After 18 hours in serum-free medium, the cells were treated with different amounts of rhEGF (Promega) for 24 hours. Erlotinib (10 µmol/L) was added 2 hours before EGF treatment.

**Western blot analyses.** Cells were lysed as reported (32). Lysates (30 or 100 µg) were resolved on 10% polyacrylamide gel and transferred to nitrocellulose membranes (GE Healthcare, Piscataway, NJ). Membranes were blocked in 5% non-fat milk in Tris-buffered saline (TBS) containing 0.1% Tween 20 (TBS-T) for 1 hour at room temperature, incubated overnight with relevant antibodies, washed, and probed with species-specific secondary antibodies coupled to horseradish peroxidase, and detected by enhanced chemiluminescence (Amersham Biosciences).

**Proliferation assays.** Cell proliferation was determined by measurement of cellular ATP levels with a high sensitivity cell proliferation/cytotoxicity kit (Vialight Plus, Cambrex Bio Sci Rockland Inc., Rockland, ME). Briefly, cells were plated in 96-well plates at a density of 1 x 10⁴ per well and exposed 24 hours later to various concentrations of erlotinib (0.1-10 µmol/L) in serum-free media. After 24 hours nucleotide (ATP) releasing reagent (100 µl) was added to each well and the plate was incubated for 10 min at room temperature. Cell lysate (180 µl) was transferred to a luminescence compatible plate. The 96-well plates were read using a Perkin Elmer LS 50B luminometer. ATP levels in cells were normalized to levels in untreated control cultures.

**Small interfering RNA treatment.** Cells in exponential growth phase were transfected with 100 nmol/L hMena-specific pooled small interfering RNA (siRNA) duplexes (siENA SMART pool) or control non-specific siRNA (Dharmacon, Lafayette, CO) using LipofectAMINE 2000 reagent (Invitrogen, Paisley, United Kingdom). After culturing for 48 hours, cells were serum deprived for 18 hours and then differently treated for Western blot analysis or proliferation assays.

**Two-dimensional electrophoresis.** Cells were washed, lyophilized, and proteins solubilised with two-dimensional electrophoresis buffer [9 mol/L urea, 10 mmol/L Tris, 4% CHAPS, 65 mmol/L DTT, 2% IPG buffer ampholine (pH 3-10), protease inhibitor cocktail]. Protein samples (250 µg) were applied to 7-cmIPG strips pH 3-10 nonlinear (Amersham Biosciences) and isoelectrofocusing was done with an IPGphor system (Amersham Biosciences) following a standard protocol as described (36). Strips were equilibrated in 50 mmol/L Tris-HCl buffer (pH 8.8) containing 6 mol/L urea, 30% glycerol, 2% SDS, and 2% DTT, followed by an incubation in the same buffer replacing DTT with 2.5% iodoacetamide. The strips were loaded on top of 10% acrylamide SDS-PAGE gels for the second dimension separation. Proteins were electrontransferred onto nitrocellulose membranes and Western blot was done as described above. Images were acquired at high resolution and two-dimensional immunoreactivity patterns analyzed using Progenesis PG240 v2005 software (Nonlinear Dynamics, Newcastle, United Kingdom). Relative molecular mass (*M*ᵣ) was estimated by comparison with *M*ᵣ reference markers (Precision, Bio-Rad, Hercules, CA) and isoelectric point (p*I*) values assigned to detected spots by calibration as described in the Amersham Biosciences guidelines.

**Patients and tissue specimens.** A series of 26 patients (median age 62 yrs; range 39-78) who underwent pancreatic resection or biopsy at the Regina Elena National Cancer Institute between 2002 and 2005 with a diagnosis of pancreatic adenocarcinoma were retrospectively collected for immunohistochemical studies. This series included 12 primary (9 stage II, 1 stage III, 2 stage IV) and 14 metastatic carcinomas (11 liver metastasis, 3 other abdominal sites). Tumors were staged according to the Union Internationale Contre le Cancer TNM System 2002 and collected according to the Internal Ethic Committee guidelines.

**Immunohistochemistry.** Pancreatic cancer specimens were fixed for 18-24h in buffered formaldehyde and then processed through to paraffin wax. hMena expression was evaluated by immunohistochemistry using the mAb clone 21 (BD Transduction, San Jose, CA; 2.5 µg/mL) that recognizes all the hMena isoforms and does not cross-react with other members of Ena/VASP family proteins (32). Dewaxing, antigen retrieval, incubation with the primary antibody, chromogenic reaction with 3,3'-diaminobenzidine (DAB) and counterstaining with Mayer Haematoxylin were performed with an automatic autostainer (Vysion Biosystems Bond, Menarini, Florence, Italy). Sections were mounted in aqueous mounting medium (Glycergel, DakoCytomation). The intensity of hMena staining, detected in the cytoplasm, was scored from 0 to 3+ according to the following criteria: no staining, score 0; weak cytoplasmic staining of neoplastic cells, score 1+; moderate cytoplasmic staining score 2+; strong cytoplasmic staining, score 3+. Evaluation of the immunohistochemical data was done independently and in blinded manner by two investigators.

### Protocol for the preparation of polyclonal antibodies

Immunogenic peptide coupled with keyhole limpet hemocyanin was injected into two rabbits by subcutaneous injection with Freund's adjuvant. The immunization was performed by five successive injections at intervals of one week. Rabbit sera were collected before the first immunization and 3 days after the fifth injection. IgG were collected from sera by precipitation in 20% ammonium sulphate and then purified by affinity chromatography using standard procedures. Specificity of the antibodies has been evaluated by Western blot using lysates of hMena^{+11a} positive and negative cells and hMena^{+11a} transfected cells.

**Statistical analysis.** All experiments were repeated at least thrice. Statistical significance was determined by Student's *t* test (two tailed) comparison between two groups of data. Asterisks indicate significant differences of experimental groups compared with the corresponding control condition (* *P* < 0.05; ** *P* < 0.01). Statistical analysis was done using GraphPad Prism 4, V4.03 software (GraphPad, Inc., San Diego, CA). Change in the phosphorylation status was evaluated, using Progenesis v.2004 software (Nonlinear Dynamics), by absorbance indicated as normalized spot volume. Normalization was done by multiplying the total spot volume by the constant factor 100, which produces spot percentage volume. Densitometric quantitation of hMena immunoreactivity was determined by ImageJ (http://rsb.info.nih.gov/ij/) and normalized in comparison with the actin immunoreactivity.

### EXAMPLE 1: hMena and hMena^{+11a} isoform expression in pancreatic cancer-derived cell lines.

To acquire insights into the expression, modulation and function of the hMena and its isoform in pancreatic cancer, the hMena and hMena^{+11a} expression was first characterized in a panel of eight pancreatic cancer cell lines by Western blot analysis. Using an anti-hMena antibody recognizing all isoforms (pan-hMena) it was observed (Figure 1A) that hMena was consistently expressed at different level in all the tumour cell lines tested. Since hMena and hMena^{+11a} isoforms are not distinguishable by Western blot because they co migrate (88-90 kDa), an anti-hMena^{+11a} antibody that specifically recognize this isoform has been used. hMena^{+11a} was selectively expressed in four out of the eight cell lines (L3.6pl, BxPC3, T3M4 and PACA44) and in the HPDE normal cell line (data not shown), whereas it was undetectable in PT45, Panc1, MiaPaCa-2 and Hs766T cell lines. Furthermore, a two-dimensional Western blot analysis was conducted on protein extracts from two representative cell lines, BxPC3 and Panel. In BxPC3 two distinct sets of spots with slightly different molecular mass and p*I* ranging between 5.4 to 6 (appearing as lower protein spots) and 5.8 to 6.2 (appearing as upper protein spots) was revealed by pan-hMena. These two set of spots correspond to the two different isoforms, hMena and hMena^{+11a} as previously reported in breast cancer (34). A different pattern was observed in Panc1 cells, which like BxPC3 cells expressed the 5.4 to 6 pI set of spots (hMena). However, the set of spots corresponding to hMena^{+11a} were absent, and a new set of protein spots displaying a lower molecular weight and more basic pI (range, 5.9-6.7) was present. Since expression of hMena^{+11a} appears to be restricted to cells with an epithelial phenotype, we evaluated markers of epithelial to mesenchymal transition (EMT) in our panel of pancreatic cancer cell lines by Western blot analysis. As shown in Figure 1C, E-cadherin was highly expressed in all of the hMena^{+11a} positive cell lines (L3.6pl, BxPC3, T3M4 and PACA44) and was absent in the hMena^{+11a} negative cell lines. Conversely, we detected expression of the mesenchymal marker vimentin in PT45, Panc1 and MiaPaCa-2 and N-cadherin in MiaPaCa-2 and Hs766T suggesting that hMena^{+11a} is a marker of an epithelial phenotype in pancreatic cancer cell lines.

### Example 2: hMena^{+11a} isoform expression correlates with sensitivity to EGFR inhibition in pancreatic cancer cell lines.

Recently we have shown that in breast cancer hMena may couple tyrosine kinase signalling to the actin cytoskeleton. In view of the role of EGFR as a relevant therapeutic target in the treatment of pancreatic cancer patients, we evaluated the growth inhibitory effect of the EGFR tyrosine-kinase inhibitor erlotinib in our panel of pancreatic cancer cell lines by exposing them to increasing concentrations (0-10 µmol/L) of the drug. As shown in Figure 2, we observed a significant heterogeneity in drug responsiveness. Considering the average steady-state plasma concentrations in erlotinib treated patients, we divided our panel in sensitive (L3.6pl, BxPC3, T3M4 and PACA44), displaying at least a 50% inhibition of proliferation at concentrations of erlotinib ≤ 1 µmol/L, and resistant (PT45, Panel, MiaPaCa-2 and Hs766T) cell lines, in which the growth rate was not significantly affected even with an erlotinib concentration of 10 µmol/L (37, 38). Of interest, all the erlotinib-sensitive cell lines expressed hMena^{+11a} indicating that this isoform identifies a specific cell phenotype in which EGFR-tyrosine kinase inhibition significantly affect cell proliferation. No hMena^{+11a} expression was in fact observed in the erlotinib-resistant cell lines. To evaluate whether the expression of other EGFR family members might affect the responsiveness of tumour cells to EGFR kinase inhibitors we analyzed the levels of EGFR family members in our panel of pancreatic cancer cell lines by Western blot (Figure 3). The expression of HER family members was not correlated with hMena and hMena^{+11a} and no correlation was found with erlotinib sensitivity, confirming previous results. A constitutive EGFR phosphorylation was observed exclusively in the sensitive pancreatic cancer cell lines hMena^{+11a} positive, consistent with our previous findings that EGFR-mediated signalling networks are "on" in the erlotinib-sensitive cells, driven by availability of the autocrine ligand production.

### Example 3: Effect of EGF and erlotinib treatment on hMena expression in pancreatic cancer cell lines.

To further test the hypothesis that hMena isoforms are along the EGFR-signalling pathway, the effects of EGF have been explored and erlotinib treatment on hMena expression in BxPC3, erlotinib-sensitive, and Panc1, erlotinib-resistant cell lines. Twenty-four hours treatment with two different EGF concentrations (50 and 100ng/ml) clearly increased hMena and hMena^{+11a} protein level as detected by Western blot analysis in both cell lines. Furthermore, the addition of erlotinib to the EGF-treated cell lines down regulated hMena expression only in the hMena^{+11a} positive BxPC3 cell line.

### Example 4: hMena knock-down reduces proliferation, AKT and MAPK activation in the erlotinib-sensitive pancreatic cancer cell lines.

In view of hMena^{+11a} role on the proliferative activity in breast cancer cells, the hMena expression in hMena^{+11a} positive, erlotinib-sensitive BxPC3 and in hMena^{+11a} negative and erlotinib-resistant Panc1 cell lines via RNA interference has been transiently knocked down, with high efficiency. In parallel, constitutive AKT and MAPK phosphorylation levels have been analyzed and it has been found that they were high in both the cell lines, consistent with the general relevance of these pathways in driving proliferation and survival in pancreatic cancer cells. However, whereas AKT and MAPK phosphorylation were strongly reduced in hMena-silenced BxPC3 cells, hMena knock-down did not or slightly affect constitutive AKT and MAPK phosphorylation in Panc1 cells. The effects of hMena knock-down on these pathways were associated with a significant reduction of the baseline growth rate in BxPC3 (45% vs 100% p=0.002) compared with the growth rate of untransfected cells and cells transfected with a control non specific siRNA. In the Panc1 cell line hMena knock-down also reduced proliferation rates but the effects were much less dramatic (78% vs 100%, p=0.01) (Figure 4). Notably, combined exposure to the hMena siRNA construct (48 h) plus erlotinib (100 nM, 24 h) resulted in an additive decrease in proliferation in the BxPC3 cells (48h) but did not in the hMena^{+11a} negative Panc1 cells (Figure 4).

### Example 5: hMena expression in pancreatic cancer lesions of 26 patients.

To evaluate the *in vivo* expression of hMena we analyzed by IHC 12 primary pancreatic tumors and 14 synchronous metastases of 26 patients. As shown in Table 1 below, pan-hMena, although with various intensities, was detected in 11 out of the 12 primary tumors (92%) analyzed. More specifically, 3 cases (25%) showed a 3+, score and 8 cases (66.6%) a 2+ score whereas only one tumour was negative.

**Table 1. hMena protein expression in invasive and metastatic pancreatic carcinoma by immunohistochemistry**

| | **hMena score** | |
|---|---|---|
| No cases | 0/1+(%) | 2+/3+(%) |
| Primary tumors: 12 | 1 (8.4) | 11 (91.6) |
| Metastatic tumors: 14 | 2 (14.2) | 12 (85.8) |

Am whereas 2 cases (14.2%) were negative. Representative cases of hMena expression are shown in Figure 5.

### Example 6: hMena^{+11a} expression correlates with an epithelial phenotype

In breast cancer the hMena^{+11a} expression exclusively characterizes breast cancer cell lines with an epithelial phenotype, expressing the epithelial marker E-Cadherin and lacking the expression of markers of a mesenchymal phenotype such as N-Cadherin. On the contrary, highly invasive breast cancer cell lines such as MDAMB231 and BT549, expressing N-cadherin and lacking the expression of E-Cadherin do not express hMena^{+11a} Figure 6. Similar results have been obtained in other solid tumors as lung, cervix, and colon cancer.

### Example 7: hMena^{+11a} is a downstream target of EGFR and HER2 in human tumors.

In breast cancer cell lines EGF treatment promotes concomitant up-regulation of hMena and hMena^{+11a}, resulting in an increase of the fraction of phosphorylated hMena^{+11a} isoform only. These events are inhibited by the pre-treatment of the cells with the EGFR inhibitor AG1478 (Di Modugno F, DeMonte L, Balsamo M, Bronzi G, Nicotra MR, Alessio M, Jager E, Condeelis JS, Santoni A, Natali PG, Nisticò P. Molecular cloning of hMena (ENAH) and its splice variant hMena+11a: epidermal growth factor increases their expression and stimulates hMena+11a phosphorylation in breast cancer cell lines.Cancer Res. 2007 Mar 15;67(6):2657-65).
Moreover, HER2 activation induced either by EGF and NRG1 treatment, or by HER2 transfection results in hMena^{+11a} up-regulation and phosphorylation in breast cancer cell lines. On the contrary Herceptin treatment down-regulates hMena^{+11a} expression in breast cancer cell lines (Di Modugno F, Mottolese M, Di Benedetto A, Conidi A, Novelli F, Perracchio L, Venturo I, Botti C, Jager E, Santoni A, Natali PG, Nisticò P. The cytoskeleton regulatory protein hMena (ENAH) is overexpressed in human benign breast lesions with high risk of transformation and human epidermal growth factor receptor-2-positive/hormonal receptor-negative tumors. Clin Cancer Res. 2006 Mar 1;12(5):1470-8)

### SEQUENCE LISTING

**SEQ ID NO: 7**
   **MTC-1-for**
   GCTGGAATGGGAGAGAGAGCGCAGAATATC
**SEQ ID NO: 8**
   **MTC-3-for**
   CAGAGCCTGTAACTTCTAAGGCCTCTTCAAC
**SEQ ID NO: 9**
   **MTC-4-rev**
   GTCAAGTCCTTCCGTCTGGACTCCATTGGC
**SEQ ID NO: 10**
   **P1For**
   CACCATGAGTGAACAGAGTATC
**SEQ ID NO: 11**
   **P8Rev**
   CTGTTCCTCTATGCAGTATTTGAC

## Claims

1. A method for discriminating between sensitive and resistant tumours to a treatment with EGFR inhibitor drugs comprising:
- *in vitro* testing whether tumour material expresses the hMena^{+11a} splicing variant of hMena, the tumour positive to said testing being sensitive to the treatment
wherein the tumour is an epithelial tumour i.e. breast, colorectal, ovarian, pancreatic, lung cancer.

2. The method according to claims 1, wherein the tumour material is selected from a specimen of tumour tissue, tumour cells or in blood circulating tumour cells.

3. The method according to anyone of claims 1 or 2, wherein the testing of the hMena^{+11a} expression is made by detecting the hMena^{+11a} isoform protein using one or more labelled antibody or labelled fragment thereof specific for said hMena^{+11a} isoform.

4. The method according to claim 3, wherein said antibody or fragment thereof is a polyclonal or a monoclonal antibody specific for the peptide SEQ ID NO: 3 or SEQ ID NO:4 or fragments thereof.

5. The method according to claim 1, wherein the testing of the hMena^{+11a} expression is made by detecting the hMena^{+11a} mature transcription products by hybridization with a labelled probe specific for hMena^{+11a}, said probe having sequence selected from the sequence SEQ ID NO:1 or a portion thereof, comprising the sequence SEQ ID NO: 2 or a portion thereof.

6. The method according to claim 1, wherein the testing of the hMena^{+11a} expression is made by detecting the hMena^{+11a} mature transcription products by PCR amplification of the hMena^{+11a} variant.

7. The method according to claim 6, wherein the amplification is made using primers amplifying a region of hMena^{+11a} comprising at least a portion of the sequence SEQ ID NO: 2.

8. The method according to claim 7, wherein the amplification is made by RT-PCR using labelled primers specific for the hMena^{+11a} variant

9. A method for detecting in the blood circulating tumoral cells of tumours sensitive to EGFR inhibitor drugs comprising:
- *in vitro* testing whether the particulate portion of sample shows expression the hMena^{+11a} splicing variant of hMena, the portion positive to said testing comprising circulating tumoral cells wherein the tumour is an epithelial tumour i.e. breast, colorectal, ovarian, pancreatic, lung cancer.

10. The *in vitro* use of a) at least an antibody or fragment thereof specific for hMena^{+11a} splicing isoform, said antibody or fragments capable of being detected; b) one or more labelled probes specific for hMena^{+11a} mature transcription products; c) two or more primers amplifying the hMena^{+11a} variant or a region of hMena^{+11a} comprising at least a portion of the sequence SEQ ID NO:2 for identifying tumours and/or blood circulating tumour cells sensitive to a treatment with EGFR inhibitor drugs, wherein the tumour is an epithelial tumour i.e. breast, colorectal, ovarian, pancreatic, lung cancer.

11. Use according to claim 10 wherein the antibody or fragment thereof is a polyclonal or monoclonal antibody specific for the sequence SEQ ID NO: 4 or its fragments.

12. Use according to claim 10 wherein said labelled probe has sequence SEQ ID NO:1 or a portion thereof comprising at least part of the sequence SEQ ID NO:2.

13. Use according to claim 10, wherein the primers amplify the hMena^{+11a} variant or a region of hMena^{+11a} are labelled primers.

## Patentansprüche

1. Verfahren zur Unterscheidung zwischen Tumoren, die gegenüber einer Behandlung mit EGFR-Hemmer Medikamenten empfindlich oder resistent sind, das umfasst:
- *In-vitro*-Prüfung, ob ein Tumormaterial die hMena^{+11a}-Spleißvariante von hMena exprimiert, wobei der Tumor, der positiv auf die Prüfung reagiert, gegenüber der Behandlung empfindlich ist,
wobei der Tumor ein epithelialer Tumor ist, zum Beispiel Brust-, Darm-, Eierstock-, Bauchspeicheldrüsen-, Lungenkrebs.

2. Verfahren gemäß Anspruch 1, wobei das Tumormaterial aus einer Probe von Tumorgewebe, Tumorzellen oder Tumorzellen, die im Blut zirkulieren, ausgewählt wird.

3. Verfahren gemäß einem der Ansprüche 1 oder 2, wobei die Prüfung der hMena^{+11a}-Exprimierung durch eine Detektion des hMena^{+11a}-Isoformproteins unter Verwendung eines oder mehrerer markierter Antikörper oder markierter Fragmente davon, die für die hMena^{+11a}-Isoform spezifisch sind, durchgeführt wird.

4. Verfahren gemäß Anspruch 3, wobei der Antikörper oder das Fragment davon ein polyklonaler oder ein monoklonaler Antikörper sind, die für das Peptid SEQ ID NO: 3 oder SEQ ID NO:4 oder Fragmenten davon spezifisch sind.

5. Verfahren gemäß Anspruch 1, wobei die Prüfung der hMena^{+11a}-Exprimierung durch eine Detektion der ausgereiften Transkriptionsprodukte durch Hybridisierung mit einer markierten Sonde, die für hMena^{+11a} spezifisch ist, durchgeführt wird, wobei die Sonde über eine Sequenz verfügt, die unter den folgenden ausgewählt wird: der Sequenz SEQ ID NO:1 oder einem Teil davon, die Sequenz SEQ ID NO:2 oder einen Teil davon umfassend.

6. Verfahren gemäß Anspruch 1, wobei die Prüfung der hMena^{+11a}-Exprimierung durch eine Detektion des ausgereiften hMena^{+11a}-Transkriptionsproduktes durch PCR-Amplifikation der hMena^{+11a}-Variante durchgeführt wird.

7. Verfahren gemäß Anspruch 6, wobei die Amplifikation unter Verwendung von Primern durchgeführt wird, die einen Bereich von hMena^{+11a} amplifizieren, der mindestens einen Teil der Sequenz SEQ ID NO:2 umfasst.

8. Verfahren gemäß Anspruch 7, wobei die Amplifikation durch RT-PCR unter Verwendung von markierten Primern durchgeführt wird, die für die hMena^{+11a}-Variante spezifisch sind.

9. Verfahren zur Detektion von im Blut zirkulierenden Tumorzellen von Tumoren, die gegenüber EGFR-Hemmer Medikamenten empfindlich sind, das umfasst:
- *In-vitro*-Prüfung, ob der besondere Teil einer Probe eine Exprimierung der hMena^{+11a}-Spleißvariante von hMena zeigt, wobei der Teil, der positiv auf die Prüfung reagiert, zirkulierende Tumorzellen umfasst, wobei der Tumor ein epithelialer Tumor ist, zum Beispiel Brust-, Darm-, Eierstock-, Bauchspeicheldrüsen-, Lungenkrebs.

10. *In-vitro*-Verwendung von a) mindestens einem Antikörpers oder einem Fragment davon, der oder das für hMena^{+11a}-Spleißisoform spezifisch ist, wobei der Antikörper oder Fragmente in der Lage sind, detektiert zu werden; b) einer oder mehreren markierten Sonden, die für ausgereifte hMena^{+11a}-Transkriptionsprodukte spezifisch sind; c) einem oder mehreren Primern, die die hMena^{+11a}-Variante oder einen Bereich von hMena^{+11a} amplifizieren, die mindestens einen Teil der Sequenz SEQ ID NO:2 umfassen, zur Identifizierung von Tumoren und/oder im Blut zirkulierenden Tumorzellen, die gegenüber einer Behandlung mit EGFR-Hemmer Medikamenten empfindlich sind, wobei der Tumor ein epithelialer Tumor ist, zum Beispiel Brust-, Darm-, Eierstock-, Bauchspeicheldrüsen, Lungenkrebs.

11. Verwendung gemäß Anspruch 10, wobei der Antikörper oder das Fragment davon ein polyklonaler oder monoklonaler Antikörper ist, der für die Sequenz SEQ ID NO:4 oder ihren Fragmenten spezifisch ist.

12. Verwendung gemäß Anspruch 10, wobei die markierte Sonde über die Sequenz SEQ ID NO:1 oder einen Teil davon verfügt, die oder der mindestens einen Teil der Sequenz SEQ ID NO:2 umfassen.

13. Verwendung gemäß Anspruch 10, wobei die Primer, die die hMena^{+11a}-Variante oder einen Bereich von hMena^{+11a} amplifizieren, markierte Primer sind.

## Revendications

1. Procédé permettant de faire la distinction entre des tumeurs sensibles et des tumeurs résistantes à un traitement par des médicaments inhibiteurs du récepteur EGFR, lequel procédé comporte le fait de faire un test *in vitro* pour savoir si le matériau tumoral exprime le variant d'épissage hMena^{+11a} de hMena, étant entendu qu'une tumeur positive audit test est sensible au traitement, la tumeur étant une tumeur épithéliale, c'est-à-dire un cancer du sein, des ovaires ou du pancréas ou un cancer colorectal ou pulmonaire.

2. Procédé conforme à la revendication 1, dans lequel le matériau tumoral est choisi parmi un échantillon de tissu tumoral, des cellules tumorales, et des cellules tumorales circulantes dans le sang.

3. Procédé conforme à la revendication 1 ou 2, dans lequel on réalise le test d'expression de hMena^{+11a} en détectant la protéine isoforme hMena^{+11a} à l'aide d'un ou de plusieurs anticorps marqué(s) ou fragment(s) d'anticorps marqué(s), spécifique(s) de ladite isoforme hMena^{+11a}.

4. Procédé conforme à la revendication 3, dans lequel ledit anticorps ou fragment d'anticorps est un anticorps monoclonal ou polyclonal, spécifique du peptide de Séquence N° 3 ou de Séquence N° 4 ou de fragments de ces peptides.

5. Procédé conforme à la revendication 1, dans lequel on réalise le test d'expression de hMena^{+11a} en détectant les produits matures de transcription de hMena^{+11a} par hybridation avec une sonde marquée spécifique de hMena^{+11a}, laquelle sonde présente une séquence choisie parmi la Séquence N° 1 et une partie de cette séquence qui comprend la Séquence N° 2 ou une partie de celle-ci.

6. Procédé conforme à la revendication 1, dans lequel on réalise le test d'expression de hMena^{+11a} en détectant les produits matures de transcription de hMena^{+11a} par amplification par PCR du variant hMena^{+11a}.

7. Procédé conforme à la revendication 6, dans lequel on réalise l'amplification à l'aide d'amorces amplifiant une région de hMena^{+11a} qui comprend au moins une partie de la Séquence N° 2.

8. Procédé conforme à la revendication 7, dans lequel on réalise l'amplification par PCR en temps réel, à l'aide d'amorces marquées spécifiques du variant hMena^{+11a}.

9. Procédé permettant de détecter dans le sang des cellules tumorales circulantes issues de tumeurs sensibles à des médicaments inhibiteurs du récepteur EGFR, lequel procédé comporte le fait de faire un test *in vitro* pour savoir s'il y a, dans la fraction particulaire de l'échantillon, expression du variant d'épissage hMena^{+11a} de hMena, étant entendu qu'une fraction positive audit test comprend des cellules tumorales circulantes, la tumeur étant une tumeur épithéliale, c'est-à-dire un cancer du sein, des ovaires ou du pancréas ou un cancer colorectal ou pulmonaire.

10. Utilisation *in vitro*
a) d'au moins un anticorps ou fragment d'anticorps spécifique de l'isoforme d'épissage hMena^{+11a}, lequel ou lesquels anticorps ou fragment(s) peut ou peuvent être détecté(s),
b) d'une ou de plusieurs sonde(s) marquée(s) spécifique(s) de produits matures de transcription de hMena^{+11a},
c) ou de deux amorces ou plus amplifiant le variant hMena^{+11a} ou une région de hMena^{+11a} comprenant au moins une partie de la Séquence N° 2,
pour identifier des tumeurs et/ou des cellules tumorales circulantes dans le sang, sensibles à un traitement par des médicaments inhibiteurs du récepteur EGFR, la tumeur étant une tumeur épithéliale, c'est-à-dire un cancer du sein, des ovaires ou du pancréas ou un cancer colorectal ou pulmonaire.

11. Utilisation conforme à la revendication 10, dans laquelle l'anticorps ou le fragment d'anticorps est un anticorps monoclonal ou polyclonal, spécifique du peptide de Séquence N° 4 ou de fragments de ce peptide.

12. Utilisation conforme à la revendication 10, dans laquelle ladite sonde marquée présente la Séquence N° 1 ou une partie de cette séquence qui comprend au moins une partie de la Séquence N° 2.

13. Utilisation conforme à la revendication 10, dans laquelle les amorces amplifiant le variant hMena^{+11a} ou une région de hMena^{+11a} sont des amorces marquées.


### SEQUENCE LISTING

<110> Istituti Fisioterapici Ospitalieri (IFO) - Istituto Regina Elena Per Lo Studio e La Cura Dei Tumori (IFO)
<120> Human Mena Isoforms Serve as Markers of Epithelial to Mesenchymal Transition and sensitivity to EGFR Inhibition in Human Cancer Cells
<130> BW498R
<160> 11
<170> PatentIn version 3.3
<210> 1
   <211> 1776
   <212> DNA
   <213> Homo sapiens
<400> 1
   atgagtgaac agagtatctg tcaggcaaga gctgctgtga tggtttatga tgatgccaat    60
   aagaagtggg tgccagctgg tggctcaact ggattcagca gagttcatat ctatcaccat   120
   acaggcaaca acacattcag agtggtgggc aggaagattc aggaccatca ggtcgtgata   180
   aactgtgcca ttcctaaagg gttgaagtac aatcaagcta cacagacctt ccaccagtgg   240
   cgagatgcta gacaggtgta tggtctcaac tttggcagca aagaggatgc caatgtcttc   300
   gcaagtgcca tgatgcatgc cttagaagtg ttaaattcac aggaaacagg gccaacattg   360
   cctagacaaa actcacaact acctgctcaa gttcaaaatg gcccatccca agaagaattg   420
   gaaattcaaa gaagacaact acaagaacag caacggcaaa aggagctgga gcgggaaagg   480
   ctggagcgag aaagaatgga aagagaaagg ttggagagag agaggttaga aagggaaagg   540
   ctggagaggg agcgactgga acaagaacag ctggagagag agagacaaga acgggaacgg   600
   caggaacgcc tggagcggca ggaacgcctg gagcggcagg aacgcctgga gcggcaggaa   660
   cgcctggatc gggagaggca agaaagacaa gaacgagaga ggctggagag actggaacgg   720
   gagaggcaag aaagggagcg acaagagcag ttagaaaggg aacagctgga atgggagaga   780
   gagcgcagaa tatcaagtgc tgctgcccct gcctctgttg agactcctct aaactctgtg   840
   ctgggagact cttctgcttc tgagccaggc ttgcaggcag cctctcagcc ggccgagact   900
   ccatcccaac agggcattgt cttgggacca cttgcacctc cacctcctcc accactccca   960
   ccagggcctg cacaggcttc agtagccctc cctcctcccc cagggccccc tccacctcct  1020
   ccactcccat ccaccgggcc tccaccgccc cctcctcccc ctcctctccc taatcaagta  1080
   ccccctcctc ctccaccacc tcctgcccca cccctccctg catctggatt ctttttggca  1140
   tccatgtcag aagacaatcg ccctttaact ggacttgcag ctgcaattgc cggagcaaaa  1200
   cttaggaaag tgtcacggat ggaggatacc tctttcccaa gtggagggaa tgctattggt  1260
   gtgaactccg cctcatctaa aacagataca ggccgtggaa atggacccct tccctttagg  1320
   ggtagtggtt taatggaaga aatgagtgcc ctgctggcca ggaggagaag aattgctgaa  1380
   aagggatcaa caatagaaac agaacaaaaa gaggacaaag gtgaagattc agagcctgta  1440
   acttctaagg cctcttcaac aagtacacct gaaccaacaa gaaaaccttg ggaaagaaca  1500
   aatacaatga atggcagcaa gtcacctgtt atctccagac gggattctcc aaggaaaaat  1560
   cagattgttt ttgacaacag gtcctatgat tcattacaca gaccaaaatc cacaccctta  1620
   tcacagccca gtgccaatgg agtccagacg gaaggacttg actatgacag gctgaagcag  1680
   gacattttag atgaaatgag aaaagaatta acaaagctaa aagaagagct cattgatgca  1740
   atcaggcagg aactgagcaa gtcaaatact gcatag                            1776
<210> 2
   <211> 63
   <212> DNA
   <213> Homo sapiens
<400> 2
   acgggattct ccaaggaaaa atcagattgt ttttgacaac aggtcctatg attcattaca 60
   cag 63
<210> 3
   <211> 21
   <212> PRT
   <213> Homo sapiens
<400> 3
   Arg Asp Ser Pro Arg Lys Asn Gln Ile Val Phe Asp Asn Arg Ser Tyr
   1 5 10 15
   Asp Ser Leu His Arg
   20
<210> 4
   <211> 18
   <212> PRT
   <213> Homo sapiens
<400> 4
   Arg Asp Ser Pro Arg Lys Asn Gln Ile Val Phe Asp Asn Arg Ser Tyr
   1 5 10 15
   Asp Ser
<210> 5
   <211> 130
   <212> DNA
   <213> Artificial
<220>
   <223> probe overlapping juncture between exon 11 and exon 11a
<400> 5
   aaccaacaag aaaaccttgg gaaagaacaa atacaatgaa tggcagcaag tcacctgtta 60
   tctccagacg ggattctcca aggaaaaatc agattgtttt tgacaacagg tcctatgatt 120
   cattacacag 130
<210> 6
   <211> 141
   <212> DNA
   <213> Artificial
<220>
   <223> probe overlapping junction between exon 11a and exon 12
<400> 6
   acgggattct ccaaggaaaa atcagattgt ttttgacaac aggtcctatg attcattaca 60
   cagaccaaaa tccacaccct tatcacagcc cagtgccaat ggagtccaga cggaaggact 120
   tgactatgac aggctgaagc a 141
<210> 7
   <211> 30
   <212> DNA
   <213> Artificial
<220>
   <223> MTC-1 forward
<400> 7
   gctggaatgg gagagagagc gcagaatatc 30
<210> 8
   <211> 31
   <212> DNA
   <213> Artificial
<220>
   <223> MTC-3 forward
<400> 8
   cagagcctgt aacttctaag gcctcttcaa c 31
<210> 9
   <211> 30
   <212> DNA
   <213> Artificial
<220>
   <223> MTC-4 reverse
<400> 9
   gtcaagtcct tccgtctgga ctccattggc 30
<210> 10
   <211> 22
   <212> DNA
   <213> Artificial
<220>
   <223> P1 forward
<400> 10
   caccatgagt gaacagagta tc 22
<210> 11
   <211> 24
   <212> DNA
   <213> Artificial
<220>
   <223> P8 reverse
<400> 11
   ctgttcctct atgcagtatt tgac 24